# EUROPÄISCHE PATENTANMELDUNG

(11) **EP 4 122 439 A1**
(43) Veröffentlichungstag der Anmeldung: **25.01.2023**
(21) Anmeldenummer: 21187445.8
(22) Anmeldetag: 23.07.2021
(51) Int. Cl.: A61J 1/00, B65D 1/00, C03C 17/30, B65D 23/02, A61K 36/00

(54) **VERWENDUNG VON SILIKONISIERTEN GLASBEHÄLTERN FÜR FLÜSSIGE PFLANZENEXTRAKTE**

(71) Anmelder: Bionorica SE, 92318 Neumarkt (DE)
(72) Erfinder: Habon, Ilona, 92367 Pilsach (DE); Ferstl, Matthias, 92331 Parsberg (DE); Reher, Markus, 61440 Oberursel (DE)
(74) Vertreter: Simandi, Claus

(57) **Zusammenfassung**

Die vorliegende Erfindung betrifft die Verwendung eines verschließbaren Glasbehälters zur Aufbewahrung von flüssigen Pflanzenextrakten, wobei die Glasinnenwand silikonisiert ist.

## Beschreibung

Die vorliegende Erfindung betrifft die Verwendung eines verschließbaren Glasbehälters zur Aufbewahrung von flüssigen Pflanzenextrakten, wobei die Glasinnenwand silikonisiert ist.

Arzneipflanzen enthalten, ggfs. in bestimmten Pflanzenteilen wie Wurzeln, Blättern, Blüten oder Früchten angereichert, Inhaltsstoffe mit pharmakologischer Wirkung und bilden die Grundlage für eine beachtliche Anzahl von Mitteln, wie Arzneimittel und Nahrungsergänzungsmittel. Zur Gewinnung dieser Inhaltsstoffe gibt es unterschiedliche Verfahren, von denen die meisten nach dem Prinzip einer wie auch immer gearteten Extraktion einschließlich Mazeration oder Perkolation der Pflanzen mit einem geeigneten Extraktionsmittel bzw. Lösungsmittel arbeiten, zumeist jedoch Alkohol oder Wasser-Alkohol und infolgedessen eine mehr oder weniger selektive Lösung und Anreicherung bestimmter pflanzlicher Wirkstoffe oder Wirkstoffgruppen in dem Extraktionsmittel bzw. Auszug ergeben. Gemäß WO 2021/019097 A1 der Anmelderin können mittels Membranfitration alkohol-freie flüssige Pflanzenextrakte bereitgestellt werden.

Solche Pflanzenextrakte können flüssig, halbfest oder fest und ein Trockenextrakt (Extracta sicca) sein, wobei z.B. der Extraktionsrückstand, der erhaltene Fluidextrakt (Extracta fluida) als Überstand bzw. die erhaltene Tinktur (Tincturae) zur Trockene eingeengt wird. Eine Trocknung kann z.B. mittels Wirbelschichttrocknung oder mittels Einengung zu einem Dickoder Spissumextrakt (zähflüssiger Extrakt, Extracta spissa) erfolgen mit anschließender Vakuumbandtrocknung oder Hordentrocknung, siehe auch z.B. EP 0 753 306 B1 der Anmelderin.

Schaubild:

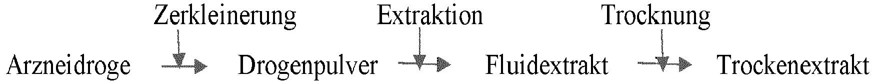

Es werden im Rahmen dieser Erfindung sogenannte flüssige Pflanzenextrakte betrachtet.

Selbstverständlich können ebenfalls die genannten Trockenextrakte wieder in einen flüssigen Pflanzenextrakt überführt werden.

So genannte standardisierte Pflanzenextrakte werden innerhalb zulässiger Grenzen auf einen vorgegebenen Gehalt an wirksamkeitsbestimmenden Inhaltsstoffen (Leitsubstanzen) eingestellt. Die Einstellung kann durch Mischen von Extraktchargen erfolgen und / oder durch Zugabe von Hilfsstoffen (siehe z.B. Ph. Eur. Monographien).

So genannte quantifizierte Pflanzenextrakte werden auf einen definierten Bereich von wirksamkeitsmitbestimmenden Inhaltsstoffen (Leitsubstanzen) eingestellt. Die Einstellung kann durch Mischen von Extraktchargen erfolgen.

Zumeist werden sogenannte Fluidextrakte als flüssige Pflanzenextrakte in galenische Formen, wie Tropfen und Säfte eingebracht.

Beispielsweise vertreibt die Anmelderin Pflanzenextrakte in der galenischen Form von Tropfen und Säfte der Marken Imupret^{®}, Sinupret^{®}, Canephron^{®} oder Bronchipret^{®}.

Flüssige Pflanzenextrakte sind jedoch hinsichtlich der Stabilität eine Herausforderung. Die physikalische und chemische Instabilität sowie die Empfindlichkeit für mikrobiologische Veränderungen dieser Pflanzenextrakte ist allgemein bekannt. Diese Instabilität kann zu Trübungen, Ausfällungen, Farbänderung, pH-Wert-Änderung und zu unterschiedlichen Abbauprozessen der enthaltenen Komponenten führen. Dadurch kann die Haltbarkeit bzw. Stabilität der flüssigen Pflanzenextrakte, insbesondere bei langer Aufbewahrung eingeschränkt sein.

In der pharmazeutischen Technologie wird die Stabilität von flüssigen pflanzlichen Arzneimitteln zumeist durch spezielle technologische Schritte (z. B. Filtration, Entkeimung, Begasung mit Schutzatmosphäre) und durch sorgfältige Auswahl einer Galenik oder Formulierung (z. B. Hilfsstoffe für Erhöhung der Löslichkeit der Komponenten, Erhöhung der Viskosität, Zusatz von Antioxidantien und Konservierungsmitteln, etc.) erreicht. Gleiche Vorgehensweisen findet man im Bereich von Lebensmitteln und Medizinprodukten.

Nach wie vor gilt es solche flüssigen Pflanzenextrakte in einer ausreichenden Stabilität einem Verbraucher zur Verfügung stellen.

Es ist bekannt, dass die Auswahl des Primärpackmittels die Haltbarkeit bzw. Stabilität von flüssigen Pflanzenextrakten beeinflusst. Weitestgehend inerte Gummi- und Kunststoffmaterialien mit strengen Vorgaben bezüglich auslösbarer Bestandteile und Glas sind die am häufigsten eingesetzten Packmaterialien.

Die Glasqualität sollte mindestens Hydrolyseklasse III (siehe Ph. Eur. 3.2.1) entsprechen. In den meisten Fällen wird Braunglas eingesetzt, um zusätzlich Lichtschutz für den Inhalt zu gewährleisten. Bevorzugte Gläser sind zumeist nicht abschließend Neutralglas, insbesondere Natronkalk-Silicatglas.

Die Gläser sind jedoch nicht vollständig inert. Sie können Metallionen abgeben, die in den Formulierungen katalytisch Abbaureaktionen auslösen können. Ebenfalls können Fehlstellen der Oberfläche chemische Reaktionen katalysieren und als Keimstelle für Kristallisation dienen.

Die Silikonisierung von Gläsern, insbesondere Glasbehältern für pharmazeutische Produkte ist im Stand der Technik beschrieben. Zumeist ist eine Silikonisierung erforderlich für Dosiereinrichtungen, deren Funktion durch Bewegung von Teilen des Packmittels ausgelöst werden können (wie z. B. Fertigspritzen). Die Silikonisierung ermöglicht das notwendige Gleiten der Packmittelkomponenten.

Die (Innen-)Silikonschicht kann temporär (Befilmung) oder dauerhaft mittels Einbrennsilikonisierung oder Beschichtungsverfahren, wie die chemische Gasphasenabscheidung (chemical vapour deposition, CVD) auf eine Glasoberfläche aufgebracht werden. Die unterschiedlichen Herstellungsmethoden sind dem Fachmann bekannt (Diss. T. Mundry, Humboldt-Universität, Berlin, 1999).

Die Einbrennsilikonisierung liefert zumeist eine homogene, ca. 15 - 50 nm dünne hydrophobe Schicht und deckt die initial vorhandene Glasoberfläche und vorhandene Fehlstellen ab (Reuter B., Petersen C., Die Silikonisierung von Spritzen, TechnoPharm2, Nr.4, 238-244 (2012)). Infolgedessen wird eine reduzierte Wechselwirkung zwischen Packmittel und Produkt erreicht.

DE 10 2009 021 501 B4 offenbart die Aufbewahrung von med. geeigneten Pflanzenextrakten in Silikonbeuteln [0031, 0018, Patentansprüche 19 und 21]. Allerdings wird ein innensilikonisierter Glasbehälter nicht offenbart, und zudem können solche Silikonbeutel bei längerer Lagerung reißen oder Feuchtigkeit anziehen.

Es besteht ein hohes Bedürfnis solche flüssigen Pflanzenextrakte mit verbesserter Stabilität bereitzustellen. Daher besteht die Aufgabe die Stabilität von flüssigen Pflanzenextrakten zu verbessern, insbesondere deren Aufbewahrung, insbesondere über lange Zeiträume, wie Monate und Jahre, so dass insbesondere die Wirksamkeit als Arzneimittel oder Nahrungsergänzungsmittel der flüssigen Pflanzenextrakte erhalten bleibt.

Überraschender Weise konnten die Erfinder feststellen, dass flüssige Pflanzenextrakte, insbesondere als Arzneimittel oder Nahrungsergänzungsmittel, in Gegenwart oder Kontakt von silikonisiertem Glas als Packmittel im Fall der Aufbewahrung eine deutliche Verbesserung der physikalischen Stabilität, insbesondere sowohl eine deutliche Reduktion der Trübung oder Ausfällung als auch pH-Stabilität als auch eine Verzögerung der chemischen Abbaureaktionen erreichen.

Daher betrifft die Erfindung die Verwendung von einem verschließbaren, silikonisierten Glasbehälter zur Aufbewahrung von mindestens einem flüssigen Pflanzenextrakt.

Im Rahmen dieser Erfindung ist ein silikonisierter Glasbehälter ein solcher, dessen Innenwand eine Silikonschicht aufweist. Der Begriff Innenwand umfasst ebenfalls einen Boden, ggfs. samt Öffnung. Beispielsweise kann eine Silikonschicht mittels einem Alkylchlorsilan - Gemisch auf das Glas aufgebracht werden. Weiterhin können Silikonöle eingesetzt werden. Ebenfalls können Silanole, Silandiole oder Silantriole im Gemisch eingesetzt werden.

Der silikonisierte Glasbehälter umfasst erfindungsgemäß keine Spritze, sondern einen verschließbaren Glasbehälter, insbesondere einseitig verschließbar, welcher mittels eines Deckels oder Aufsatzes an einer Öffnung verschlossen werden kann. Ein geeigneter Verschluss ist z.B. ein Drehverschluss, Aufsatz oder Druckaufsatz. Der Aufsatz kann z.B. eine Dosierungseinheit aufweisen. Weiterhin sind solche Glasbehälter erfindungsgemäß umfasst, welche für eine topische, orale, parenterale oder nasale Applikation geeignet sind, und eine entsprechend geeignete Austrittsöffnung aufweisen (z.B. Flasche mit Drehverschluss, Flasche mit Pumpapplikator, Nasenspray, Nasentropfen, etc.). Der silikonisierte Glasbehälter kann mit mindestens einem flüssigen Pflanzenextrakt, ggfs. samt Hilfs- und Zusatzstoffen zur Aufbewahrung oder Lagerung befüllt werden.

Die besagte Aufbewahrung des flüssigen Pflanzenextrakts kann besonders vorteilhaft bei verbesserter Stabilität (siehe Beispiele) drei Monate, vorzugsweise 6 Monate oder 12 Monate oder länger erfolgen.

Im Rahmen dieser Erfindung wird unter einem "Pflanzenextrakt" ein Vielkomponentengemisch aus Naturstoffen verstanden, welches mehr als zwei Naturstoffe, insbesondere mehr als 10 oder 100 Naturstoffe, insbesondere mehr als 200, 300, 500 oder 1.000 Naturstoffe enthält. Pflanzenextrakte können beispielsweise mittels Extraktion, Perkolation oder Mazeration aus Pflanzenmaterialien gewonnen werden. Als Extraktionsmittel können alkoholhaltige Lösungsmittel, C1-C5-Alkohole, Ethanol verwendet werden. Eine übliche Extraktion ist beispielsweise eine wässrig-ethanolische Extraktion, insbesondere ein Gemisch aus Wasser / Ethanol (50:50, 70:30, 30:70) z.B. bei 15 bis 80 Grad Celsius und Normaldruck. Der Begriff Pflanzenextrakt umfasst insbesondere Fluidextrakte (Extractum fluidum), wobei eine flüssige Drogenzubereitung bereitgestellt wird, bei der für die Extraktion der Droge vorzugsweise möglichst wenig Extraktionsflüssigkeit eingesetzt wird. Dies wirkt sich auf das Drogen-Extrakt-Verhältnis (DEV) aus.

Ein flüssiger Pflanzenextrakt im Sinne dieser Erfindung bedeutet, dass der Pflanzenextrakt zum überwiegenden Teil oder vollständig in einem oder mehreren Lösungsmitteln, ggfs. samt Hilfs- und Zusatzstoffen, gelöst vorliegt.

Die Pflanzenextrakte können nicht abschließend aus solchen Pflanzenmaterialien erhalten werden, solche wie Achillea, Aloe, Althaea, Angelica, Arnika, Artemisia, Cannabis, Capsicum, Carum, Caulophyllum, Centaurium, Chelidonium, Cimicifuga, Cnicus, Citrus, Crataegus, Cyclamen, Cynara, Echinacea, Equisetum, Glycyrrhiza, Guaiacum, Hedeara, Humulus, Iberis, Iris, Juglans, Lavandula, Levisticum, Lilium, Matricaria, Melissa, Mentha, Basilicum (Ocimum), Passiflora, Pelargonium, Phytolacca, Pimpinella, Primula, Potentilla, Punica, Quercus, Rosmarinus, Rumex, Salix, Salvia, Sambucus, Silybum, Strychnos, Taraxacum, Thymus, Vaccinium, Valeriana, Vebena, Vitex, Vitis.

Die Pflanzenextrakte können nicht abschließend aus solchen Pflanzenmaterialien erhalten werden, solche wie Achillea millefolium Aloe Vera, Althaea officinalis, Angelica archangelica, Arnika montana, Artemisia vulgaris, annua und absinthium, Cannabis indica, Cannabis ruderalis, Cannabis sativa, Capsicum annuum, Carum carvi, Caulophyllum thalictroides, Centaurium, Chelidonium majus, Cimicifuga racemose, Cnicus benedictus Citrus reticulata, Citrus sinensis, Citrus junos, Citrus medica, Citrus maxima, Citrus aurantifolia, Citrus aurantium, Citrus hystrix, Citrus limon, Citrus paradisi, Cistus incanus, Crataegus, Cyclamen purpurascens, Cynara cardunculus, Echinacea purpurea und angust, Equisetum arvense, Glycyrrhiza glabra, Glycyrrhiza inflata, Glycyrrhiza uralensis, Guaiacum officinale und sanctum, Hedeara helix, Humulus lupulus, Iberis amara, Juglans regia, Lavandula angustifolia, Levisticum officinale, Lilium tigrinum, Matricaria chamomilla, Melissa officinalis, Mentha candensis, Mentha arvensis, Mentha piperita, Ocimum basilicum, Passiflora, Phytolacca americana, Pelargonium sidoides, Pimpinella anisum, Primula veris, elatior und vulgaris, Potentilla anserina, Punica granatum, Quercus robur, Quercus petraea, Quercus pubescens, Rosmarinus, Rumex cripus, Rumex obtusifolius, Rumex alpinus, Rumex patientia, Rumex acetosa, Rumex acetosella, Rumex thyrsiflorus, Salix purpurea, Salix daphnoides, Salix fragilis, Salvia officinalis, Sambucus nigra, Silybum marianum, Strychnos ignatii, Taraxacum officinale, Thymus vulgaris, Vaccinium macrocarpon, Vaccinium myrtillus, Valeriana officinalis, Vebena officinalis, Vitex agnus castus, Vitis vinifera.

In einer weiteren Ausführungsform der Erfindung kann eine vorteilhafte Vor- oder Nachbehandlung der Pflanzenextrakte erfolgen, solche wie Entkeimung mittels Kurzzeiterhitzung, Pasteurisierung, Ultrahocherhitzung, entkeimende Filtration, o. ä.. Ebenso ist eine Konservierung mit Konservierungsmitteln, wie z.B. Kaliumsorbat möglich.

Der besagte Pflanzenextrakt wird in flüssiger Form bereitgestellt, insbesondere in einer Arzneiform oder galenischen Form ausgewählt aus der Gruppe flüssiger Zubereitung, Tropfen, Saft, Sirup, Aufguss, insbesondere Rachenspray sowie Desinfektionslösungen, Nasenspray, flüssige Präparate für die Inhalation, Spüllösungen, insbesondere in Kombination mit physiologischen und hyperosmolaren Konzentrationen von Salzen oder Salzgemischen, bevorzugt Kochsalz, insbesondere Meersalz.

Daher kann der mindestens eine flüssige Pflanzenextrakt in einer Zubereitung vorliegen, umfassend weitere Hilfs- und Zusatzstoffe.

### Beispiele:

Die folgenden Beispiele und Abbildungen sollen die Erfindung näher erläutern, ohne jedoch die Erfindung zu beschränken.

### Beispiel 1:

### Trübung der flüssigen Pflanzenextrakte und Ausfällungen

Eine Entwicklungscharge eines alkoholfreien Pflanzenextraktes aus fünf Pflanzen (Sambucus, Gentianae, Verbena, Rumex, Primula wie z.B. in Sinupret^{®} Saft) wurde nach der Herstellung in Flaschen mit und ohne Innensilikonisierung abgefüllt. Nach 6 Monaten Lagerzeit bei der Lagerbedingung 40°C/75% r. F. wurde die Trübung der zwei Proben verglichen. Die Probe, die in den Flaschen ohne Innensilikonisierung gelagert wurde, zeigt eine deutliche Trübung, die Lösung in der Flasche mit Innensilikonisierung ist weiterhin klar.

Ein Entwicklungsversuch mit anderer pflanzlicher Zusammensetzung(alkoholhaltiger Extrakt aus Thymus und Hedera wie z. B. in Bronchipret^{®} Tropfen) zeigt nach 12 Monaten Lagerung bei Raumtemperatur (RT) und 25°C/60% r. F. deutliche Ausfällungen im Packmittel ohne Innensilikonisierung). Der Niederschlag bildet eine gut erkennbare Schicht am Flaschenboden. In den Flaschen mit Innensilikonisierung ist ein Niederschlag nicht erkennbar.

### Beispiel 2:

### Veränderung der Zusammensetzung der Produkte

### pH-Wert

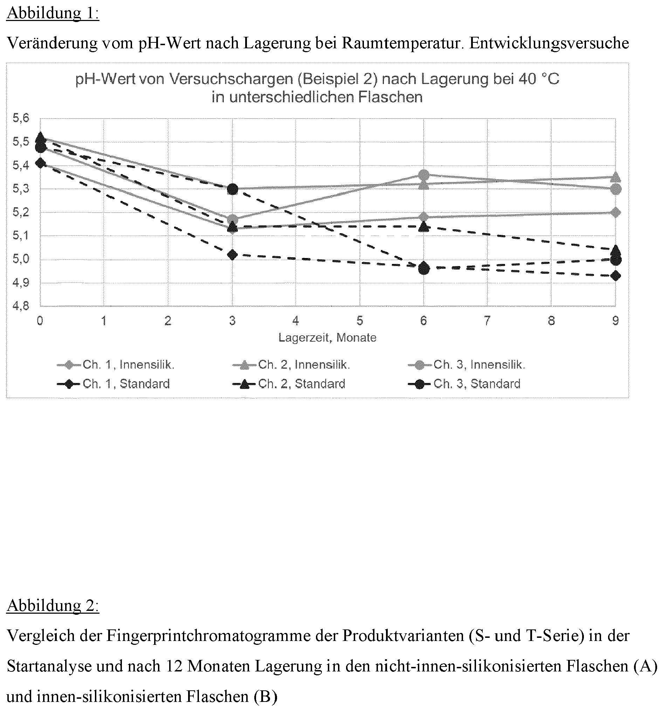

Der pH-Wert der Zubereitungen kann großen Einfluss auf die chemische Stabilität haben. Versuchschargen eines alkoholhaltigen Pflanzenextraktes aus fünf Pflanzen (Sambucus, Gentianae, Verbena, Rumex, Primula wie z. B. in Sinupret Tropfen) zeigen in einem Packmittel ohne Innensilikonisierung einen pH-Abfall. Die Veränderung der gleichen Chargen, in innen-silikonisierten Flaschen gelagert, ist kleiner (siehe Abb. 1).

### Chromatographische Fingerprints

Die Fingerprintchromatogramme der pharmazeutischen Versuche werden für die Bewertung der Haltbarkeit herangezogen. Dafür wird die Veränderung der Anzahl und der relativen Intensität der erfassten Zonen in Dünnschicht-Chromatogrammen mit dem Fingerprint aus der Ausgangsanalyse abgeglichen.

In den DC-Chromatogrammen einer Versuchsreihe der Produktfamilie (Bronchipret Saft und Tropfen) wurde die Auswirkung unterschiedlicher Primärpackmittel auf die Veränderung des Fingerprintes untersucht. Die Methode erfasst Polyphenole (z. B. Flavonoide).

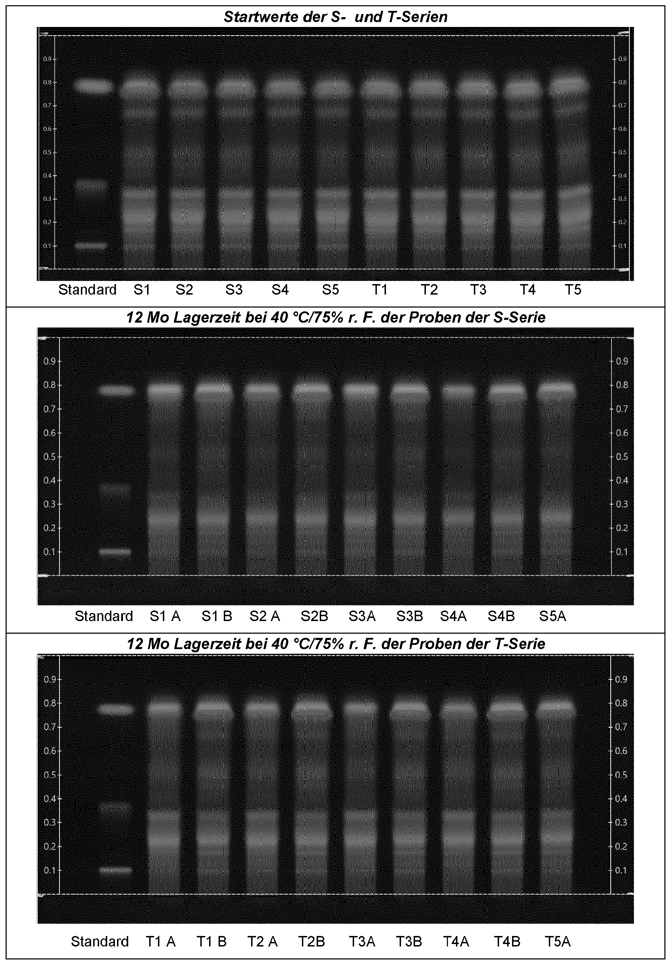

Jeweils 5 Produktvarianten (S- und T-Serie) wurden aus den gleichen Fluidextrakt-Chargen hergestellt. Die Varianten wurden unterschiedlich hergestellt (unterschiedlich filtriert) um die Auswirkung dieser Schritte auf die Haltbarkeit zu untersuchen. Alle Produktvarianten wurden in unterschiedlichen Flaschen abgefüllt und gelagert. Nach 12 Monaten Lagerzeit ist der Fingerprint der Proben, die in innen-silikonisierten Flaschen (B) gelagert waren, weniger verändert als in den nicht-innen-silikonisierten Flaschen (A) wie z.B. Glasart III (Abb. 2).

Besonders deutliche Unterschiede gibt es in beiden Serien bei den folgenden Zonen (R_{f}-Wert ist auf der y-Achse in Abb. 2 aufgetragen):

| | |
|---|---|
| R_{f} 0,1 | in innen-silikoniserten Flaschen (B) überall sichtbar, |
| R_{f} 0,2 | in innen-silikoniserten Flaschen (B) überall sichtbar, |
| R_{f} 0,7 | in innen-silikoniserten Flaschen (B) überall sichtbar, |
| R_{f} 0,8 | in innen-silikoniserten Flaschen (B) überall sichtbar. |

### Diskussion

Die Verwendung von innen-silikonisierten Glasbehältnissen erhöht die physikalische und chemische Stabilität von flüssigen Pflanzenextrakten die als flüssige pharmazeutische Zubereitungen in Verkehr gebracht werden.

Die Abbauprozesse von unterschiedlichen Inhaltsstoffen in flüssigen Pflanzenextrakten und deren Zubereitungen sind bei Verwendung von innen-silikonisierten Glasbehältnissen reduziert.

## Patentansprüche

1. Verwendung von einem verschließbaren, silikonisierten Glasbehälter zur Aufbewahrung von mindestens einem flüssigen Pflanzenextrakt.

2. Verwendung nach Anspruch 1, wobei der verschließbare, silikonisierte Glasbehälter eine Innenwand mit einer Silikonschicht aufweist.

3. Verwendung nach einem der Ansprüche 1 bis 2, wobei der verschließbare, silikonisierte Glasbehälter einseitig verschließbar ist, insbesondere durch einen Deckel, Aufsatz, Druckaufsatz oder Pumpapplikator.

4. Verwendung nach einem der Ansprüche 1 bis 3, wobei der verschließbare, silikonisierte Glasbehälter einen Drehverschluss aufweist.

5. Verwendung nach einem der Ansprüche 1 bis 4, wobei der verschließbare, silikonisierte Glasbehälter eine Innenwand mit einer Silikonschicht aufweist, welche durch Einbrennsilikonisierung oder Gasphasenabscheidung (CVD) hergestellt wird.

6. Verwendung nach einem der Ansprüche 1 bis 5, wobei die Aufbewahrung mindestens drei Monate, 6 Monate oder 12 Monate oder länger erfolgt.

7. Verwendung nach einem der Ansprüche 1 bis 6, wobei der mindestens eine flüssige Pflanzenextrakt aus einer Gattung ausgewählt ist aus der Gruppe Achillea, Aloe, Althaea, Angelica, Arnika, Artemisia, Cannabis, Capsicum, Carum, Caulophyllum, Centaurium, Chelidonium, Cimicifuga, Cnicus, Citrus, Crataegus, Cyclamen, Cynara, Echinacea, Equisetum, Glycyrrhiza, Guaiacum, Hedeara, Humulus, Iberis, Iris, Juglans, Lavandula, Levisticum, Lilium, Matricaria, Melissa, Mentha, Basilicum (Ocimum), Passiflora, Pelargonium, Phytolacca, Pimpinella, Primula, Potentilla, Punica, Quercus, Rosmarinus, Rumex, Salix, Salvia, Sambucus, Silybum, Strychnos, Taraxacum, Thymus, Vaccinium, Valeriana, Vebena, Vitex, Vitis.

8. Verwendung nach einem der Ansprüche 1 bis 7, wobei der mindestens eine Pflanzenextrakt aus einer Art ausgewählt ist aus der Gruppe Achillea millefolium Aloe Vera, Althaea officinalis, Angelica archangelica, Arnika montana, Artemisia vulgaris, annua und absinthium, Cannabis indica, Cannabis ruderalis, Cannabis sativa, Capsicum annuum, Carum carvi, Caulophyllum thalictroides, Centaurium, Chelidonium majus, Cimicifuga racemose, Cnicus benedictus Citrus reticulata, Citrus sinensis, Citrus junos, Citrus medica, Citrus maxima, Citrus aurantifolia, Citrus aurantium, Citrus hystrix, Citrus limon, Citrus paradisi, Cistus incanus, Crataegus, Cyclamen purpurascens, Cynara cardunculus, Echinacea purpurea und angust, Equisetum arvense, Glycyrrhiza glabra, Glycyrrhiza inflata, Glycyrrhiza uralensis, Guaiacum officinale und sanctum, Hedeara helix, Humulus lupulus, Iberis amara, Juglans regia, Lavandula angustifolia, Levisticum officinale, Lilium tigrinum, Matricaria chamomilla, Melissa officinalis, Mentha candensis, Mentha arvensis, Mentha piperita, Ocimum basilicum, Passiflora, Phytolacca americana, Pelargonium sidoides, Pimpinella anisum, Primula veris, elatior und vulgaris, Potentilla anserina, Punica granatum, Quercus robur, Quercus petraea, Quercus pubescens, Rosmarinus, Rumex cripus, Rumex obtusifolius, Rumex alpinus, Rumex patientia, Rumex acetosa, Rumex acetosella, Rumex thyrsiflorus, Salix purpurea, Salix daphnoides, Salix fragilis, Salvia officinalis, Sambucus nigra, Silybum marianum, Strychnos ignatii, Taraxacum officinale, Thymus vulgaris, Vaccinium macrocarpon, Vaccinium myrtillus, Valeriana officinalis, Vebena officinalis, Vitex agnus castus, Vitis vinifera.

9. Verwendung nach einem der Ansprüche 1 bis 8, wobei der mindestens eine flüssige Pflanzenextrakt in einer Zubereitung vorliegt, umfassend weitere Hilfs- und Zusatzstoffe.

10. Verwendung nach einem der Ansprüche 1 bis 9, wobei der mindestens eine flüssige Pflanzenextrakt in einer galenischen Form ausgewählt ist aus der Gruppe flüssiger Zubereitungen, Tropfen, Saft, Sirup, Aufguss, Rachenspray sowie Desinfektionslösungen, Nasenspray, flüssige Präparate für die Inhalation, Spüllösungen, insbesondere in Kombination mit physiologischen und hyperosmolaren Konzentrationen von Salzen oder Salzgemischen, bevorzugt Kochsalz, insbesondere Meersalz.
